# EUROPEAN PATENT SPECIFICATION

(11) **EP 2 802 340 B1**
(45) Date of publication and mention of the grant of the patent: **28.10.2020**
(21) Application number: 13701320.7
(22) Date of filing: 11.01.2013
(51) Int. Cl.: A61K 9/00, A61K 38/15, A61K 9/20, A61K 9/19, A61K 47/26, A61P 35/00

(54) **ROMIDEPSIN FORMULATIONS AND USES THEREOF**
ROMIDEPSINFORMULIERUNGEN UND IHRE VERWENDUNGEN
FORMULATIONS DE LA ROMIDEPSINE ET UTILISATIONS DE CELLE-CI

(30) Priority: 12.01.2012 US 201261586066 P
(43) Date of publication of application: 19.11.2014
(73) Proprietor: Celgene Corporation, Summit, NJ 07901 (US)
(72) Inventor: HUANG, Lianfeng, Basking Ridge, NJ 07920 (US); HUI, Ho-wah, Basking Ridge, NJ 07920 (US); PEYKOV, Victor, San Diego, CA 92128 (US); FOSS, Willard, R., San Diego, CA 92130 (US); NARINGREKAR, Vijay, Princeton, NJ 08540 (US); LAI, Mei, Longmont, CO 80504 (US)
(74) Representative: Jones Day
(86) International application number: PCT/US2013/021212
(87) International publication number: WO 2013/106696

(56) References cited:
- WO-A1-2006/129105
- WO-A1-2006/129105
- WO-A2-2007/146234
- WO-A2-2008/127659
- WO-A2-2008/127659
- US-A1- 2007 148 228

## Description

### FIELD

Provided herein are formulations of romidepsin. Also provided are methods for producing these formulations and uses thereof.

### BACKGROUND

Cancer is a major public health problem in the United States and in the world. Currently, one in 4 deaths in the United States is due to cancer. Each year, the American Cancer Society estimates the numbers of new cancer cases and deaths expected in the United States in the current year and compiles the most recent data on cancer incidence, mortality, and survival based on incidence data from the National Cancer Institute, the Centers for Disease Control and Prevention, and the North American Association of Central Cancer Registries and mortality data from the National Center for Health Statistics. A total of 1,596,670 new cancer cases and 571,950 deaths from cancer were projected to occur in the United States in 2011. Overall cancer incidence rates were stable since late 1990s. The reduction in the overall cancer death rates since 1990 in men and 1991 in women translated to the avoidance of about 898,000 deaths from cancer. Despite an obvious progress, approximately 560,000 people died of cancer in 2006 in the United States alone. Aging of the general population and development of new forms of cancer contribute to the problem.

Romidepsin has been shown to have anticancer activities. The drug is approved in the U.S. for treatment of cutaneous T-cell lymphoma (CTCL) and peripheral T-cell lymphoma (PTCL), and is currently being tested, for example, for use in treating patients with other hematological malignancies (*e.g,* , multiple myeloma, *etc.*) and solid tumors (*e.g.,* prostate cancer, pancreatic cancer, *etc.*)*.* It is thought to act by selectively inhibiting deacetylases (*e.g*., histone deacetylase, tubulin deacetylase), promising new targets for development of a new class of anti-cancer therapies (Nakajima et al., Experimental Cell Res 241:126-133, 1998). One mode of action involves the inhibition of one or more classes of histone deacetylases (HDAC).

As cancer remains a major worldwide public health problem, there is a continued need for effective therapies to treat cancer.

### SUMMARY

In one embodiment, provided herein is a romidepsin formulation. according to claim 1. In one embodiment, the formulation comprises an excipient. Excipients suitable for the formulations provided herein include, but are not limited to, antiadherents, binders, coatings, disintegrants, fillers and diluents, flavours, colours, lubricants, glidants, preservatives, sorbents, and sweeteners. In one embodiment, the excipient is diluent. The diluent is mannitol.

In one embodiment, provided herein are formulations according to any of claims 1 to 10 for use in methods to treat proliferative diseases. In some embodiments, provided herein are methods to treat cancer. In some embodiments, cancers include, but are not limited to, carcinomas, sarcomas, leukemias, lymphomas and the like. In certain embodiments, the cancer is a hematological malignancy. In certain embodiments, the cancer is a solid tumor.

In one embodiment, provided are methods of producing a romidepsin formulation.

### DESCRIPTION OF THE DRAWINGS

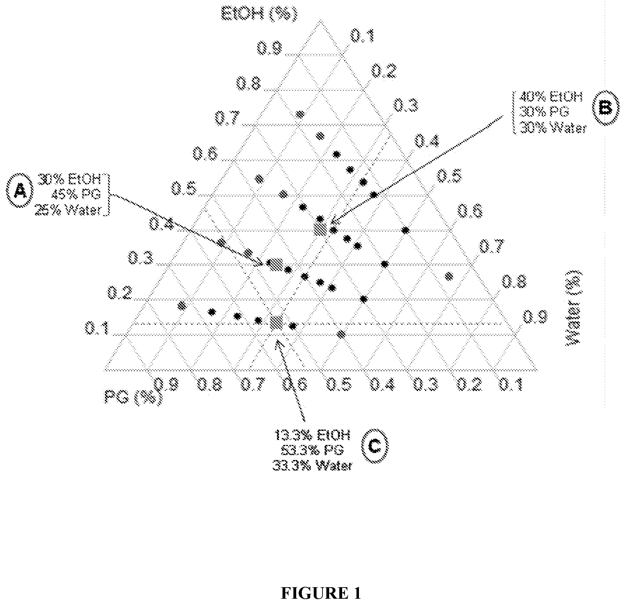

Figure 1 depicts diluent phase diagram of romidepsin mannitol formulation in 3 component solvent systems. Red dots represent the solvent system where the formulation was not dissolved in 2 mL of the solvent mixture within 3 minutes. Black dots represent the solvent system where the formulation was dissolved in 2 mL of the solvent mixture within 30 seconds. Blue squares represent various solvent systems: diluent A: 30% EtOH, 45% PG and 25% water; diluent B: 40% EtOH, 30% PG and 30% water; and diluent C: 13.1% EtOH, 53.3% PG and 33.3% water.

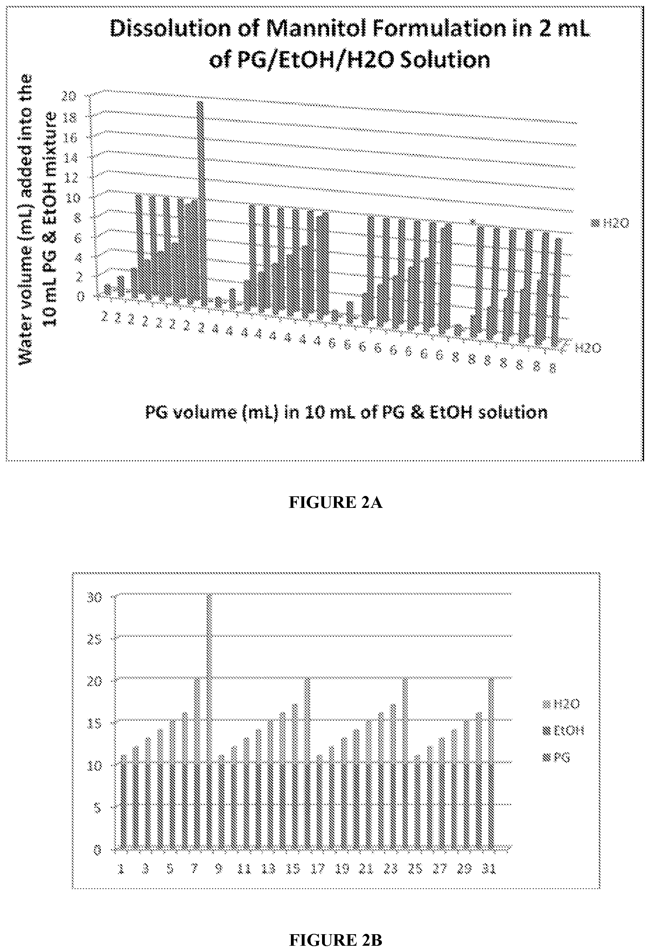

Figures 2A and 2B demonstrate dissolution of romidepsin mannitol formulation in 2 mL of PG/EtOH/H₂O solution. The X axis represents the ratios of PG and EtOH in 10 mL of the solution (2 means 2 mL of PG and 8 ml of EtOH; 4 means 4 mL of PG and 6 ml of EtOH; 6 means 6 mL of PG and 4 ml of EtOH; and 8 means 8 mL of PG and 2 ml of EtOH). The blue bars represent the volume of water added into 10 mL of PG/EtOH/H₂O solution (for example: if x = 4 and blue bar = 5, the solvent composition is 4:6:5 for PG/EtOH/H₂O).

### DETAILED DESCRIPTION

### Definitions

As used in the specification and the accompanying claims, the indefinite articles "a" and "an" and the definite article "the" include plural as well as singular referents, unless the context clearly dictates otherwise.

As used herein, and unless otherwise specified, the term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 30%, 25%, 20%, 15%, 10%, 9%, 8%, 7%, 6%, 5%, 4%, 3%, 2%, 1%, 0.5%, 0.1%, or 0.05% of a given value or range.

As used herein, and unless otherwise specified, the terms "treat," "treating" and "treatment" refer to the eradication or amelioration of a disease or disorder, or of one or more symptoms associated with the disease or disorder. In certain embodiments, the terms refer to minimizing the spread or worsening of the disease or disorder resulting from the administration of one or more prophylactic or therapeutic agents to a subject with such a disease or disorder. In some embodiments, the terms refer to the administration of a compound or dosage form provided herein, with or without one or more additional active agent(s), after the diagnosis or the onset of symptoms of the particular disease.

As used herein, and unless otherwise specified, the terms "prevent," "preventing" and "prevention" refer to the prevention of the onset, recurrence or spread of a disease or disorder, or of one or more symptoms thereof. In certain embodiments, the terms refer to the treatment with or administration of a compound or dosage form provided herein, with or without one or more other additional active agent(s), prior to the onset of symptoms, particularly to subjects at risk of disease or disorders provided herein. The terms encompass the inhibition or reduction of a symptom of the particular disease. In certain embodiments, subjects with familial history of a disease are potential candidates for preventive regimens. In certain embodiments, subjects who have a history of recurring symptoms are also potential candidates for prevention. In this regard, the term "prevention" may be interchangeably used with the term "prophylactic treatment."

As used herein, and unless otherwise specified, the terms "manage," "managing" and "management" refer to preventing or slowing the progression, spread or worsening of a disease or disorder, or of one or more symptoms thereof. Often, the beneficial effects that a subject derives from a prophylactic and/or therapeutic agent do not result in a cure of the disease or disorder. In this regard, the term "managing" encompasses treating a subject who had suffered from the particular disease in an attempt to prevent or minimize the recurrence of the disease.

As used herein, and unless otherwise specified, "amelioration" of the symptoms of a particular disorder by administration of a particular pharmaceutical composition refers to any lessening, whether permanent or temporary, lasting or transient, that can be attributed to or associated with the administration of the composition.

As used herein, and unless otherwise specified, the term "therapeutically effective amount" or "effective amount" of a compound means an amount sufficient to provide a therapeutic benefit in the treatment or management of a disease or disorder, or to delay or minimize one or more symptoms associated with the disease or disorder. A "therapeutically effective amount" or "effective amount" of a compound means an amount of therapeutic agent, alone or in combination with one or more other agent(s), which provides a therapeutic benefit in the treatment or management of the disease or disorder. The terms "therapeutically effective amount" and "effective amount" can encompass an amount that improves overall therapy, reduces, delays, or avoids symptoms or causes of disease or disorder, or enhances the therapeutic efficacy of another therapeutic agent.

As used herein, and unless otherwise specified, a "prophylactically effective amount" of a compound is an amount sufficient to prevent a disease or disorder, or prevent its recurrence. A prophylactically effective amount of a compound means an amount of therapeutic agent, alone or in combination with one or more other agent(s), which provides a prophylactic benefit in the prevention of the disease. The term "prophylactically effective amount" can encompass an amount that improves overall prophylaxis or enhances the prophylactic efficacy of another prophylactic agent.

As used herein, and unless otherwise specified, the term "subject" is defined herein to include animals such as mammals, including, but not limited to, primates (e.g., humans), cows, sheep, goats, horses, dogs, cats, rabbits, rats, mice, and the like. In specific embodiments, the subject is a human. The terms "subject" and "patient" are used interchangeably herein in reference, for example, to a mammalian subject, such as a human. In particular embodiments, a subject having cancer is a subject who has been previously diagnosed as having cancer.

As used herein, and unless otherwise specified, "neoplasm" is an abnormal mass of tissue as a result of neoplasia. The growth of neoplastic cells exceeds and is not coordinated with that of the normal tissues around it. The growth persists in the same excessive manner even after cessation of the stimuli. It usually causes a lump or tumor. Neoplasms may be benign, pre-malignant (carcinoma in situ) or malignant (cancer).

As used herein, and unless otherwise specified, "tumor" refers to all neoplastic cell growth and proliferation, whether malignant or benign, and all pre-cancerous and cancerous cells and tissues. As used herein, and unless otherwise specified, "neoplastic" refers to any form of dysregulated or unregulated cell growth, whether malignant or benign, resulting in abnormal tissue growth. Thus, "neoplastic cells" include malignant and benign cells having dysregulated or unregulated cell growth.

As used herein, and unless otherwise specified, the terms "cancer" and "cancerous" refer to or describe the physiological condition in mammals that is typically characterized by unregulated cell growth. Examples of cancer include, but are not limited to, lymphoma, leukemia, and solid tumors, such as, for example, lung cancer. In one embodiment, the term "cancer" as used herein includes, but is not limited to, solid tumors and blood-borne tumors. The term "cancer" refers to disease of skin tissues, organs, blood, and vessels, including, but not limited to, cancers of the bladder, bone or blood, brain, breast, cervix, chest, colon, endometrium, esophagus, eye, head, kidney, liver, lymph nodes, lung, mouth, neck, ovaries, pancreas, prostate, rectum, stomach, testis, throat, and uterus. Specific cancers include, but are not limited to, advanced malignancy, amyloidosis, neuroblastoma, meningioma, atypical meningioma, hemangiopericytoma, multiple brain metastase, glioblastoma multiforms, glioblastoma, brain stem glioma, poor prognosis malignant brain tumor, malignant glioma, recurrent malignant glioma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karyotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, low grade follicular lymphoma, metastatic melanoma (localized melanoma, including, but not limited to, ocular melanoma), malignant mesothelioma, malignant pleural effusion mesothelioma syndrome, peritoneal carcinoma, papillary serous carcinoma, gynecologic sarcoma, soft tissue sarcoma, scelroderma, cutaneous vasculitis, Langerhans cell histiocytosis, leiomyosarcoma, fibrodysplasia ossificans progressiva, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapy-insensitive prostate cancer, papillary thyroid carcinoma, follicular thyroid carcinoma, medullary thyroid carcinoma, and leiomyoma. In a specific embodiment, the cancer is metastatic. In another embodiment, the cancer is refractory or resistant to chemotherapy or radiation.

As used herein, and unless otherwise specified, the term "proliferative" disorder or disease refers to unwanted cell proliferation of one or more subset of cells in a multicellular organism resulting in harm (*i.e.,* discomfort or decreased life expectancy) to the multicellular organism. For example, as used herein, proliferative disorder or disease includes neoplastic disorders and other proliferative disorders.

As used herein, and unless otherwise specified, the term "relapsed" refers to a situation where a subject, that has had a remission of cancer after a therapy, has a return of cancer cells.

As used herein, and unless otherwise specified, the term "refractory" or "resistant" refers to a circumstance where a subject, even after intensive treatment, has residual cancer cells in the body.

As used herein, and unless otherwise specified, the term "drug resistance" refers to the condition when a disease does not respond to the treatment of a drug or drugs. Drug resistance can be either intrinsic, which means the disease has never been responsive to the drug or drugs, or it can be acquired, which means the disease ceases responding to a drug or drugs that the disease had previously responded to. In certain embodiments, drug resistance is intrinsic. In certain embodiments, the drug resistance is acquired.

As used herein, and unless otherwise specified, the term "anticancer agent" or "cancer therapeutic agent" is meant to include histone deacetylase (HDAC) inhibitors, including, but not limited to, romidepsin, anti-proliferative agents and chemotherapeutic agents, including, but not limited to, antimetabolites (*e.g*., 5-fluoro uracil, methotrexate, fludarabine, cytarabine (also known as cytosine arabinoside or Ara-C), and high dose cytarabine), antimicrotubule agents (*e.g*., vinca alkaloids, such as vincristine and vinblastine; and taxanes, such as paclitaxel and docetaxel), alkylating agents (*e.g*., mechlorethamine, chlorambucil, cyclophosphamide, melphalan, melphalan, ifosfamide, carmustine, azacitidine, decitabine, busulfan, cyclophosphamide, dacarbazine, ifosfamide, and nitrosoureas, such as carmustine, lomustine, bischloroethylnitrosurea, and hydroxyurea), platinum agents (*e.g.*, cisplatin, carboplatin, oxaliplatin, satraplatin (JM-216), and CI-973), anthracyclines (*e.g*., doxorubicin and daunorubicin), antitumor antibiotics (*e.g*., mitomycin, bleomycin, idarubicin, adriamycin, daunomycin (also known as daunorubicin, rubidomycin, or cerubidine), and mitoxantrone), topoisomerase inhibitors (*e.g*., etoposide and camptothecins), purine antagonists or pyrimidine antagonists (*e.g*., 6-mercaptopurine, 5-fluorouracil, cytarabine, clofarabine, and gemcitabine), cell maturing agents (*e.g*., arsenic trioxide and tretinoin), DNA repair enzyme inhibitors (*e.g*., podophyllotoxines, etoposide, irinotecan, topotecan, and teniposide), enzymes that prevent cell survival (*e.g*., asparaginase and pegaspargase), histone deacetylase inhibitors (*e.g.,* vorinostat), any other cytotoxic agents (*e.g.,* estramustine phosphate, dexamethasone, prednimustine, and procarbazine), hormones (*e.g*., dexamethasone, prednisone, methylprednisolone, tamoxifen, leuprolide, flutamide, and megestrol), monoclonal antibodies (*e.g*., gemtuzumab ozogamicin, alemtuzumab, rituximab, and yttrium-90-ibritumomab tiuxetan), immuno-modulators (*e.g*., thalidomide and lenalidomide), Bcr-Abl kinase inhibitors (*e.g*., AP23464, AZD0530, CGP76030, PD180970, SKI-606, imatinib, BMS354825 (dasatinib), AMN107 (nilotinib), and VX-680), hormone agonists or antagonists, partial agonists or partial antagonists, kinase inhibitors, surgery, radiotherapy (*e.g*., gamma-radiation, neutron bean radiotherapy, electron beam radiotherapy, proton therapy, brachytherapy, and systemic radioactive isotopes), endocrine therapy, biological response modifiers (*e.g*., interferons, interleukins, and tumor necrosis factor), hyperthermia and cryotherapy, and agents to attenuate any adverse effects (*e.g*., antiemetics).

As used herein, and unless otherwise specified, the terms "co-administration" and "in combination with" include the administration of two or more therapeutic agents simultaneously, concurrently or sequentially within no specific time limits unless otherwise indicated. In one embodiment, the agents are present in the cell or in the subject's body at the same time or exert their biological or therapeutic effect at the same time. In one embodiment, the therapeutic agents are in the same composition or unit dosage form. In other embodiments, the therapeutic agents are in separate compositions or unit dosage forms. In certain embodiments, a first agent can be administered prior to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), essentially concomitantly with, or subsequent to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapeutic agent.

As used herein, and unless otherwise specified, the terms "composition," "formulation," and "dosage form" are intended to encompass products comprising the specified ingredient(s) (in the specified amounts, if indicated), as well as any product(s) which result, directly or indirectly, from combination of the specified ingredient(s) in the specified amount(s).

As used herein, and unless otherwise specified, the term "excipient" refers to a pharmacologically inactive substance used as a carrier for the active ingredient of a medication or as a bulking agent to allow for convenient and accurate dosage of an active ingredient.

As used herein, and unless otherwise specified, the term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. In one embodiment, by "pharmaceutical" or "pharmaceutically acceptable" it is meant that any diluent(s), excipient(s) or carrier(s) in the composition, formulation, or dosage form are compatible with the other ingredient(s) and not deleterious to the recipient thereof. *See, e.g.,* Remington, The Science and Practice of Pharmacy, 21st Edition; Lippincott Williams & Wilkins: Philadelphia, PA, 2005; Handbook of Pharmaceutical Excipients, 5th Edition; Rowe et al., ed., The Pharmaceutical Press and the American Pharmaceutical Association: 2005; and Handbook of Pharmaceutical Additives, 3rd Edition; Ash and Ash ed., Gower Publishing Company: 2007; Pharmaceutical Preformulation and Formulation, Gibson ed., CRC Press LLC: Boca Raton, FL, 2004.

As used herein, and unless otherwise specified, the term "pharmaceutically acceptable salts" is meant to include salts of active compounds which are prepared with relatively nontoxic acids. Acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, et al. (1977) J. Pharm. Sci. 66:1-19).

A pharmaceutically acceptable salt form of a compound can be prepared *in situ* during the final isolation and purification of the compound, or separately by reacting the free base functionality with a suitable organic or inorganic acid. Examples of typical pharmaceutically acceptable, nontoxic acid addition salts are salts of an amino group formed with inorganic acids such as hydrochloric acid, hydrobromic acid, phosphoric acid, sulfuric acid, and perchloric acid, or with organic acids such as acetic acid, oxalic acid, maleic acid, tartaric acid, citric acid, succinic acid, or malonic acid or by using other methods used in the art such as ion exchange. Other pharmaceutically acceptable salts can include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts can include, when appropriate, nontoxic ammonium, quaternary ammonium, and amine cations formed using counterions such as halide, hydroxide, carboxylate, sulfate, phosphate, nitrate, loweralkyl sulfonate, and aryl sulfonate.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

As used herein, and unless otherwise specified, the terms, "polymorphs" and "polymorphic forms" and related terms refer to one of a variety of different crystal structures that can be adopted by a particular compound. In some embodiments, polymorphs occur when a particular chemical compound can crystallize in more than one structural arrangement. Different polymorphs may have different physical properties such as, for example, melting temperatures, heats of fusion, solubilities, dissolution rates and/or vibrational spectra as a result of the arrangement or conformation of the molecules in the crystal lattice. The differences in physical properties exhibited by polymorphs affect pharmaceutical parameters such as storage stability, compressibility and density (important in formulation and product manufacturing), and dissolution rates (an important factor in determining bioavailability). Differences in stability can result from changes in chemical reactivity (*e.g*., differential oxidation, such that a dosage form discolors more rapidly when comprised of one polymorph than when comprised of another polymorph) or mechanical changes (*e.g*., tablets crumble on storage as a kinetically favored polymorph converts to thermodynamically more stable polymorph) or both (*e.g*., tablets of one polymorph are more susceptible to breakdown at high humidity). As a result of solubility/dissolution differences, in the extreme case, some polymorphic transitions may result in lack of potency or, at the other extreme, toxicity. In addition, the physical properties of the crystal may be important in processing, for example, one polymorph might be more likely to form solvates or might be difficult to filter and wash free of impurities (*i.e.,* particle shape and size distribution might be different between one polymorph relative to the other).

As used herein, and unless otherwise specified, the term, "solvate" refers to a crystal form of a substance which contains solvent.

As used herein, and unless otherwise specified, the term "hydrate" refers to a crystal form adopted by a particular compound in which either a stoichiometric or non-stoichiometric amount of water is incorporated into the crystal lattice.

As used herein, and unless otherwise specified, the term "prodrug" refers to structurally modified forms of the compound that readily undergo chemical changes under physiological conditions to provide the compound. Additionally, prodrugs can be converted to the compound by chemical or biochemical methods in an *ex vivo* environment. Prodrugs are often useful because, in some situations, they may be easier to administer than the compound, or parent drug. They may, for instance, be bioavailable by oral administration whereas the parent drug is not. The prodrug may also have improved solubility in pharmaceutical compositions over the parent drug. A wide variety of prodrug derivatives are known in the art, such as those that rely on hydrolytic cleavage or oxidative activation of the prodrug. An example, without limitation, of a prodrug would be a compound which is administered as an ester (the "prodrug"), but then is metabolically hydrolyzed to the carboxylic acid, the active entity.

As used herein, and unless otherwise specified, the term "anhydrous" refers to a form of a compound that is substantially free of water. One of skill in the art will appreciate that an anhydrous solid can contain various amounts of residual water wherein that water is not incorporated in the crystalline lattice. Such incorporation of residual water can depend upon a compound's hygroscopicity and storage conditions.

As used herein, and unless otherwise specified, the term "isostructural" or "isostructure" refers to two or more solid forms of a compound containing essentially the same three-dimensional arrangement of geometrically similar structural units. In some embodiments, "isostructural" forms show with similar or identical unit cell dimensions, the same space group, and similar or identical atomic coordinates for common atoms. In some embodiments, "isostructural" forms have the same structure, but not the same cell dimensions nor the same chemical composition, and have comparable variability in their atomic coordinates to that of the cell dimensions and chemical composition.

As used herein, and unless otherwise specified, the term "lyophilization" or "freeze-drying" refers to a process of dehydration by freezing a material and then reducing the surrounding pressure to allow frozen water or other solvent (*e.g., tert-*BuOH) in the material to sublimate directly from the solid phase to the gas phase.

As used herein, and unless otherwise specified, the term "parenteral" includes subcutaneous, intravenous, intramuscular, intra-articular, intra-synovial, intrasternal, intrathecal, intrahepatic, intralesional and intracranial injection or infusion techniques.

As used herein, and unless otherwise specified, the term "substantially free of' means containing no more than an insignificant amount. In some embodiments, a composition or preparation is "substantially free of' a recited element if it contains less than 5%, 4%, 3%, 2%, or 1%, by weight of the element. In some embodiments, the composition or preparation contains less than 0.9%, 0.8%, 0.7%, 0.6%, 0.5%, 0.4%, 0.3%, 0.2%, 0.1% or less of the recited element. In some embodiments, the composition or preparation contains an undetectable amount of the recited element.

As used herein, and unless otherwise specified, the expression "unit dose" refers to a physically discrete unit of a formulation appropriate for a subject to be treated (*e.g.,* for a single dose); each unit containing a predetermined quantity of an active agent selected to produce a desired therapeutic effect (it being understood that multiple doses may be required to achieve a desired or optimum effect), optionally together with a pharmaceutically acceptable carrier, which may be provided in a predetermined amount. The unit dose may be, for example, a volume of liquid (e.g,. an acceptable carrier) containing a predetermined quantity of one or more therapeutic agents, a predetermined amount of one or more therapeutic agents in solid form, a sustained release formulation or drug delivery device containing a predetermined amount of one or more therapeutic agents, etc. It will be appreciated that a unit dose may contain a variety of components in addition to the therapeutic agent(s). For example, acceptable carriers (*e.g*., pharmaceutically acceptable carriers), diluents, stabilizers, buffers, preservatives, etc., may be included as described *infra.* It will be understood, however, that the total daily usage of a formulation of the present disclosure will be decided by the attending physician within the scope of sound medical judgment. The specific effective dose level for any particular subject or organism may depend upon a variety of factors including the disorder being treated and the severity of the disorder; activity of specific active compound employed; specific composition employed; age, body weight, general health, sex and diet of the subject; time of administration, and rate of excretion of the specific active compound employed; duration of the treatment; drugs and/or additional therapies used in combination or coincidental with specific compound(s) employed, and like factors well known in the medical arts.

As used herein, and unless otherwise specified, a compound described herein is intended to encompass all possible stereoisomers, unless a particular stereochemistry is specified. Where structural isomers of a compound are interconvertible *via* a low energy barrier, the compound may exist as a single tautomer or a mixture of tautomers. This can take the form of proton tautomerism; or so-called valence tautomerism in the compound.

### Romidepsin

Romidepsin is a natural product which was isolated from *Chromobacterium violaceum* by Fujisawa Pharmaceuticals (Published Japanese Patent Application Hei 7 (1995)-64872; and U.S. Patent 4,977,138, issued December 11. Various preparations and purifications of romidepsin are described in PCT Publication WO 02/20817.

Romidepsin is a bicyclic peptide consisting of four amino acid residues (D-valine, D-cysteine, dehydrobutyrine, and L-valine) and a novel acid (3-hydroxy-7-mercapto-4-heptenoic acid), which contains both amide and ester bonds. Romidepsin can be obtained from *C*. *violaceum* using fermentation. It can also be prepared by synthetic or semisynthetic means. The total synthesis of romidepsin reported by Kahn et al. (J. Am. Chem. Soc. 118:7237-7238, 1996) involves 14 steps and yields romidepsin in 18% overall yield. The structure of romidepsin is shown below (formula I):

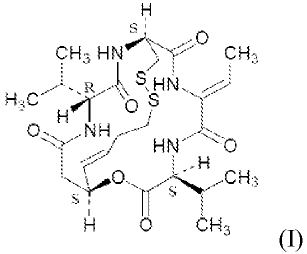

Romidepsin has been shown to have anti-microbial, immunosuppressive, and antitumor activities. In the US, it is approved for the treatment of patients with cutaneous T-cell lymphoma (CTCL) and peripheral T-cell lymphoma (PTCL). It is currently being tested for multiple myeloma and solid tumors (*e.g.,* prostate cancer, pancreatic cancer, *etc.*) and is thought to act by selectively inhibiting deacetylases (*e.g*., histone deacetylase, tubulin deacetylase) (Nakajima et al., Exp Cell Res 241:126-133, 1998). One mode of action of romidepsin involves the inhibition of one or more classes of histone deacetylases (HDAC). Preparations and purification of romidepsin is described, for example, in U.S. Patent 4,977,138 and International PCT Application Publication WO 02/20817.

Exemplary forms of romidepsin include, but are not limited to, salts, esters, prodrugs, isomers, stereoisomers (*e.g*., enantiomers, diastereomers), tautomers, protected forms, reduced forms, oxidized forms, derivatives, and combinations thereof, with the desired activity (*e.g*., deacetylase inhibitory activity, aggressive inhibition, cytotoxicity). In certain embodiments, romidepsin is a pharmaceutical grade material and meets the standards of the U.S. Pharmacopoeia, Japanese Pharmacopoeia, or European Pharmacopoeia. In certain embodiments, the romidepsin is at least 95%, at least 98%, at least 99%, at least 99.9%, or at least 99.95% pure. In certain embodiments, the romidepsin is at least 95%, at least 98%, at least 99%, at least 99.9%, or at least 99.95% monomeric. In certain embodiments, no impurities are detectable in the romidepsin materials (*e.g*., oxidized material, reduced material, dimerized or oligomerized material, side products, *etc.*)*.* Romidepsin typically includes less than 1.0%, less than 0.5%, less than 0.2%, or less than 0.1% of total other unknowns. The purity of romidepsin may be assessed by appearance, HPLC, specific rotation, NMR spectroscopy, IR spectroscopy, UV/Visible spectroscopy, powder x-ray diffraction (XRPD) analysis, elemental analysis, LC-mass spectroscopy, or mass spectroscopy.

In one embodiment, the formulation contains a derivative of romidepsin.

In one embodiment, the derivative of romidepsin is of the formula (II): 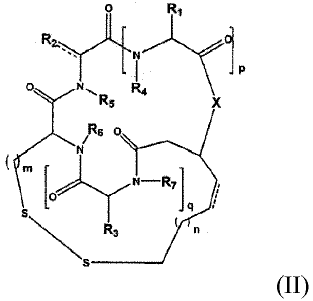 wherein
m is 1, 2, 3 or 4;
n is 0, 1, 2 or 3;
p and q are independently 1 or 2;
X is 0, NH, or NR₈;
R₁, R₂, and R₃ are independently hydrogen, unsubstituted or substituted, branched or unbranched, cyclic or acyclic aliphatic; unsubstituted or substituted, branched or unbranched, cyclic or acyclic heteroaliphatic; unsubstituted or substituted aryl; or unsubstituted or substituted heteroaryl; and R₄, R₅, R₆, R₇ and R₈ are independently hydrogen, or substituted or unsubstituted, branched or unbranched, cyclic or acyclic aliphatic; and pharmaceutically acceptable forms thereof.

In one embodiment, m is 1, n is 1, p is 1, q is 1, X is 0, R₁, R₂, and R₃ are unsubstituted or substituted, branched or unbranched acyclic aliphatic. In one embodiment, R₄, R₅, R₆ and R₇ are all hydrogen.

In one embodiment, the derivative of romidepsin is of the formula (III): 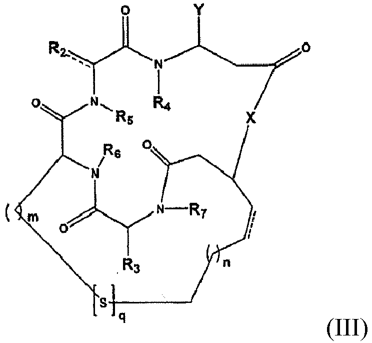 wherein:
m is 1, 2, 3 or 4;
n is 0, 1, 2 or 3;
q is 2 or 3;
X is 0, NH, or NR₈;
Y is ORB, or SR₈;
R₂ and R₃ are independently hydrogen, unsubstituted or substituted, branched or unbranched, cyclic or acyclic aliphatic, unsubstituted or substituted, branched or unbranched, cyclic or acylic heteroaliphatic, unsubstituted or substituted aryl or unsubstituted or substituted heteroaryl;
R₄, R₅, R₆, R₇ and R₈ are independently selected from hydrogen or substituted or unsubstituted, branched or unbranched, cyclic or acyclic aliphatic, and pharmaceutically acceptable forms thereof.

In one embodiment, m is 1, n is 1, q is 2, X is NH and R₂ and R₃ are unsubstituted or substituted, branched or unbranched, acyclic aliphatic. In one embodiment, R₄, R₅, R₆ and R₇ are all hydrogen.

In one embodiment, the derivative of romidepsin is of the formula (IV): 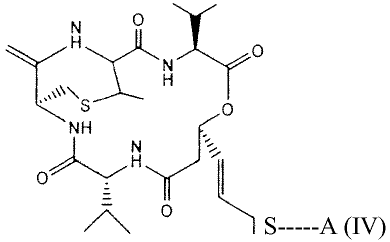 wherein:
A is a moiety that is cleaved under physiological conditions to yield a thiol group and includes, for example, an aliphatic or aromatic acyl moiety (to form a thioester bond), an aliphatic or aromatic thioxy (to form a disulfide bond), or the like, and pharmaceutically acceptable forms thereof. Such aliphatic or aromatic groups can include a substituted or unsubstituted, branched or unbranched, cyclic or acyclic aliphatic group, a substituted or unsubstituted aromatic group, a substituted or unsubstituted heteroaromatic group, or a substituted or unsubstituted heterocyclic group. A can be, for example, -COR₁, -SC(=0)-0-R₁, or -SR₂;
R₁ is independently hydrogen, substituted or unsubstituted amino, substituted or unsubstituted, branched or unbranched, cyclic or acyclic aliphatic, substituted or unsubstituted aromatic group, substituted or unsubstituted heteroaromatic group, or a substituted or unsubstituted heterocyclic group. In one embodiment, R₁ is hydrogen, methyl, ethyl, n-propyl, isopropyl, n-butyl, isobutyl, benzyl, or bromobenzyl;
R₂ is a substituted or unsubstituted, branched or unbranched, cyclic or acyclic aliphatic group, a substituted or unsubstituted aromatic group, a substituted or unsubstituted heteroaromatic group, or a substituted or unsubstituted heterocyclic group.

In one embodiment, R₂ is methyl, ethyl, 2- hydroxyethyl, isobutyl, a fatty acid, a substituted or unsubstituted benzyl, a substituted or unsubstituted aryl, cysteine, homocysteine, or glutathione.

In one embodiment, the derivatives of romidepsin are of formulae (V) or (V'): 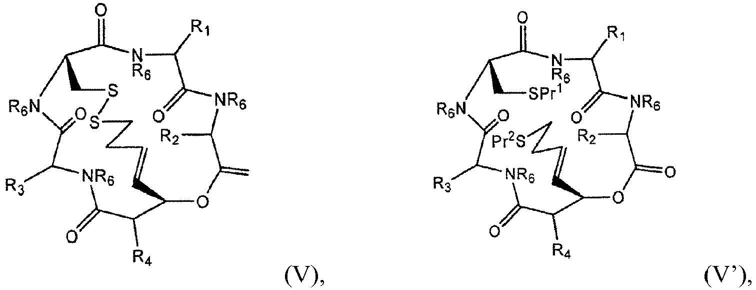 wherein:
each of R₁, R₂, R₃ and R₄ is the same or different and represent an amino acid side chain moiety;
each R₆ is the same or different and represents hydrogen or (C₁-C₄)alkyl; and
Pr¹ and Pr² are the same or different and represent hydrogen or thiol-protecting group.

In one embodiment, the amino acid side chain moieties are those derived from natural amino acids. In one embodiment, the amino acid side chain moieties are those derived from unnatural amino acids.

In one embodiment, each amino acid side chain is a moiety selected from hydrogen, (C₁-C₆)alkyl, (C₂-C₆)alkenyl, -L-O-C(0)-R', -L-C(0)-0-R", -L-A, -L-NR"R", -L-Het-C(0)-Het-R", and -L-Het-R", wherein L is a (C₁-C₆)alkylene group, A is phenyl or a 5-or 6-membered heteroaryl group, each R' is the same or different and represents (C₁-C₄)alkyl, each R" is the same or different and represent H or (C₁-C₆)alkyl, each -Het- is the same or different and is a heteroatom spacer selected from -0-, -N(R"')-, and -S-, and each R'" is the same of different and represents hydrogen or (C₁-C₄)alkyl.

In one embodiment, R₆ is hydrogen.

In one embodiment, Pr¹ and Pr² are the same or different and are selected from hydrogen and a protecting group selected from a benzyl group which is optionally substituted by (C₁-C₆)alkoxy, (C₁-C₆)acyloxy, hydroxy, nitro, picolyl, picolyl-N-oxide, anthrylmethyl, diphenylmethyl, phenyl, t-butyl, adamanthyl, (C₁-C₆)acyloxymethyl, (C₁-C₆)alkoxymethyl, tetrahydropyranyl, benzylthiomethyl, phenylthiomethyl, thiazolidine, acetamidemethyl, benzamidomethyl, tertiary butoxycarbonyl (BOC), acetyl and its derivatives, benzoyl and its derivatives, carbamoyl, phenylcarbamoyl, and (C₁-C₆)alkylcarbamoyl.

Various romidepsin derivatives of formula (V) and (V') are disclosed in PCT application publication WO 2006/129105, published December 7, 2006.

It has been found that romidepsin can exist in a variety of solid forms. Such solid forms include neat crystal forms. Such solid forms also include solvated forms and amorphous forms. The present disclosure provides certain such solid forms of romidepsin. In certain embodiments, the present disclosure provides compositions comprising romidepsin in a form described herein. In some embodiments of provided compositions, romidepsin is present as a mixture of one or more solid forms; in some embodiments of provided compositions, romidepsin is present in only a single form.

In certain embodiments, romidepsin is provided as an amorphous form. In certain embodiments, romidepsin is provided as a solvated form. Solid forms of romidepsin are described in WO 02/20817, published March 14, 2002, and in WO 2012/009336, published January 19, 2012,

In some embodiments, all of romidepsin that is present in a particular composition is present in a particular form; in some such embodiments, the composition is substantially free of any other form of romidepsin. In some embodiments, a composition comprises romidepsin present in a combination of different forms.

The present disclosure provides a lyophilate of romidepsin containing amorphous romidepsin.

In some embodiments, the present disclosure provides one or more solid forms as described herein, in combination with one or more other components. In some such embodiments, other components are selected from the group consisting of, for example, buffers, carriers, crystallization inhibitors, diluents, excipients, pH adjustors, solvents, or other pharmaceutical additives for administration to a patient.

In certain embodiments, where romidepsin is in amorphous form (*e.g.,* in certain lyophilates), such compositions comprise one or more crystallization inhibitors.

In certain embodiments, the formulation or composition comprises an amount of a crystallization inhibitor of at least about 1%, 5%, 10%, 12%, 15%, 18%, 20%, 25%, 30%, 35%, 40%, 45%, 50%, 55%, 60%, 65%, 70%, 75%, 80%, 85%, 90%, 95% or 99% (w/w), based on the total weight of the formulation or composition.

The crystallization inhibitor is mannitol. The amount of romidepsin and the amount of mannitol is present in a composition in a ratio of about 1 : 2 (by weight). In some embodiments, the present disclosure provides pharmaceutical compositions that comprise and/or are prepared from solid forms of romidepsin as described herein. In some embodiments, a pharmaceutical composition comprises a therapeutically effective amount of romidepsin and at least one pharmaceutically acceptable carrier or excipient.

In some embodiments, the present disclosure provides compositions comprising or prepared from romidepsin solid forms described herein, which compositions further comprise one or more additional components.

In some embodiments, provided compositions comprise, in addition to romidepsin at least one other component, such as a carrier (*e.g*., pharmaceutically acceptable carrier). Except insofar as any conventional carrier medium is incompatible with compounds or forms described herein, such as by producing any undesirable biological effect or otherwise interacting in a deleterious manner with any other component(s) of compositions and/or the use thereof is contemplated to be within the scope of this disclosure.

In some embodiments, materials which can serve as acceptable carriers (*e.g*., pharmaceutically acceptable carriers) include, but are not limited to, sugars such as lactose, glucose, and sucrose; starches such as corn starch and potato starch; cellulose and its derivatives such as sodium carboxymethyl cellulose, ethyl cellulose, and cellulose acetate; powdered tragacanth; malt; gelatin; talc; Cremophor; Solutol; excipients such as cocoa butter and suppository waxes; oils such as peanut oil, cottonseed oil; sunflower oil; sesame oil; olive oil; corn oil and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions; as well as other non-toxic compatible lubricants such as sodium lauryl sulfate and magnesium stearate; as well as coloring agents, releasing agents, coating agents, sweetening, flavoring and perfuming agents, preservatives and antioxidants can also be present in the composition, according to the judgment of the formulator.

### METHODS OF USE

In one embodiment, provided is a composition for use for treating, preventing, or managing cancer in a patient comprising administering to said patient an effective amount of a formulation provided herein.

In some embodiments, cancers treatable by the uses provided herein include, but are not limited to, carcinomas, sarcomas, haematological malignancies and the like. In certain embodiments, the cancer is a hematological malignancy. In certain embodiments, the cancer is a solid tumor.

In one embodiment, haematological malignancies that can be treated by the uses provided herein include, but are not limited to, lymphomas, leukemias, multiple myeloma, plasma cell-derived cancers, relapsed hematological malignancies, and refractory hematological malignancies. In one embodiment, lymphomas that can be treated by the uses provided herein include, but are not limited to, mature B-cell lymphomas, mature T-cell and natural killer cell lymphomas, Hodgkin's lymphomas and immunodeficiency-associated lymphoproliferative disorders. In another embodiment, lymphomas that can be treated by the uses provided herein include, but are not limited to, small lymphocytic lymphoma, follicular lymphoma, Mantle cell lymphoma, diffuse large B-cell lymphoma, Burkitt lymphoma, B-cell lymphoblastic lymphoma, small cleaved B-cell lymphoma, non-cleaved B-cell lymphoma, cutaneous T-cell lymphoma (CTCL), and peripheral T-cell lymphoma (PTCL). In another embodiment, leukemias that can be treated by the methods provided herein include, but are not limited to, acute lymphoid leukemia (ALL), chronic lymphocytic leukemia (CLL), acute myeloid leukemia (AML), chronic myeloid leukemia (CML), MLL-rearranged ALL, including leukemias that are relapsed, refractory or resistant to conventional therapy, multiple myeloma, and plasma cell-derived cancer.

In one embodiment, solid cancers that can be treated by the uses provided herein include, but are not limited to, cancer of the skin; lymph node; breast; cervix; uterus; gastrointestinal tract; pancreas, lung; ovary; prostate; colon; rectal; mouth; brain; head and neck; throat; testes; kidney; pancreas; bone; spleen; liver; bladder; larynx; or nasal passages, and relapsed or refractory cancer.

In one embodiment, an effective amount of romidepsin to be used is a therapeutically effective amount. In one embodiment, the amounts of romidepsin to be used in the uses provided herein include an amount sufficient to cause improvement in at least a subset of patients with respect to symptoms, overall course of disease, or other parameters known in the art. Precise amounts for therapeutically effective amounts of romidepsin in the pharmaceutical compositions will vary depending on the age, weight, disease, and condition of the patient.

### Dosing

### Standard Dosing for Romidepsin

In one embodiment, romidepsin is administered intravenously. In one embodiment, romidepsin is administered intravenously over a 1-6 hour period. In one embodiment, romidepsin is administered intravenously over a 3-4 hour period. In one embodiment, romidepsin is administered intravenously over a 5-6 hour period. In one embodiment, romidepsin is administered intravenously over a 4 hour period.

In one embodiment, romidepsin is administered in a dose ranging from 0.5 mg/m² to 28 mg/m². In one embodiment, romidepsin is administered in a dose ranging from 0.5 mg/m² to 5 mg/m². In one embodiment, romidepsin is administered in a dose ranging from 1 mg/m² to 25 mg/m². In one embodiment, romidepsin is administered in a dose ranging from 1 mg/m² to 20 mg/m². In one embodiment, romidepsin is administered in a dose ranging from 1 mg/m² to 15 mg/m². In one embodiment, romidepsin is administered in a dose ranging from 2 mg/m² to 15 mg/m². In one embodiment, romidepsin is administered in a dose ranging from 2 mg/m² to 12 mg/m². In one embodiment, romidepsin is administered in a dose ranging from 4 mg/m² to 12 mg/m². In one embodiment, romidepsin is administered in a dose ranging from 6 mg/m² to 12 mg/m². In one embodiment, romidepsin is administered in a dose ranging from 8 mg/m² to 12 mg/m². In one embodiment, romidepsin is administered in a dose ranging from 8 mg/m² to 10 mg/m². In one embodiment, romidepsin is administered in a dose of about 8 mg/m². In one embodiment, romidepsin is administered in a dose of about 9 mg/m². In one embodiment, romidepsin is administered in a dose of about 10 mg/m². In one embodiment, romidepsin is administered in a dose of about 11 mg/m². In one embodiment, romidepsin is administered in a dose of about 12 mg/m². In one embodiment, romidepsin is administered in a dose of about 13 mg/m². In one embodiment, romidepsin is administered in a dose of about 14 mg/m². In one embodiment, romidepsin is administered in a dose of about 15 mg/m².

In one embodiment, romidepsin is administered in a dose of 14 mg/m² over a 4 hour iv infusion on days 1, 8 and 15 of the 28 day cycle. In one embodiment, the cycle is repeated every 28 days.

In one embodiment, increasing doses of romidepsin are administered over the course of a cycle. In one embodiment, the dose of about 8 mg/m² followed by a dose of about 10 mg/m², followed by a dose of about 12 mg/m² is administered over a cycle.

In some embodiments, unit doses of romidepsin are within the range of about 0.5 mg/ m² to about 28 mg/m². In certain embodiments, unit doses are in the range of about 1 mg/m² to about 25 mg/m². In certain embodiments, unit doses are in the range of about 0.5 mg/ m² to about 15 mg/m². In certain embodiments, unit doses are the range of about 1 mg/ m² to about 15 mg/m². In certain embodiments, unit doses are in the range of about 1 mg/m² to about 8 mg/m². In certain embodiments, unit doses are in the range of about 0.5 mg/m² to about 5 mg/m². In certain embodiments, the unit doses are in the range of about 2 mg/m² to about 10 mg/m². In some embodiments, unit doses are in the range of about 10 mg/m² to about 20 mg/m². In certain embodiments, unit doses are in the range of about 5 mg/m² to about 10 mg/m². In some embodiments, unit doses are in the range of about 10 mg/m² to about 15 mg/m². In some embodiments, unit doses are in the range of about 6 to about 19 mg/m². In some embodiments, unit doses are approximately 8 mg/m². In still other embodiments, the unit doses are approximately 9 mg/m². In still other embodiments, unit doses are approximately 10 mg/m². In still other embodiments, unit doses are approximately 11 mg/m². In still other embodiments, unit doses are approximately 12 mg/m². In still other embodiments, unit doses are approximately 13 mg/m². In still other embodiments, unit doses are approximately 14 mg/m². In still other embodiments, unit doses are approximately 15 mg/m². In still other embodiments, unit doses are approximately 30 mg/m².

In certain embodiments, different individual unit doses within the romidepsin therapy regimen are different. In some embodiments, increasing doses of romidepsin are administered over the course of a cycle. In certain embodiments, a dose of approximately 8 mg/m² is administered, followed by a dose of approximately 10 mg/m², followed by a dose of approximately 12 mg/m² may be administered over a cycle.

An amount of romidepsin administered in individual unit doses varies depending on the form of romidepsin being administered. In certain embodiments, individual unit doses of romidepsin are administered on one day followed by several days on which romidepsin is not administered. In certain embodiments, romidepsin is administered twice a week. In certain embodiments, romidepsin is administered once a week. In other embodiments, romidepsin is administered every other week.

In some embodiments, romidepsin is administered daily (for example for 2 weeks), twice weekly (for example for 4 weeks), thrice weekly (for example for 4 weeks), or on any of a variety of other intermittent schedules (e.g., on days 1, 3, and 5; on days 4 and 10; on days 1 and 15; on days 5 and 12; or on days 5, 12, and 19 of 21 or 28 day cycles).

In certain embodiments, romidepsin is administered on days 1, 8, and 15 of a 28 day cycle. In certain particular embodiments, an 8 mg/m² dose of romidepsin is administered on day 1, a 10 mg/m² dose of romidepsin is administered on day 8, and a 12 mg/m² dose of romidepsin is administered on day 15. In certain embodiments, romidepsin is administered on days 1 and 15 of a 28 day cycle with day 8 being skipped. A 28 day dosing cycle may be repeated. In certain embodiments, a 28 day cycle is repeated 2-10, 2-7, 2-5, or 3-10 times. In certain embodiments, the treatment includes 5 cycles. In certain embodiments, the treatment includes 6 cycles. In certain embodiments, the treatment includes 7 cycles. In certain embodiments, the treatment includes 8 cycles. In certain embodiments, 10 cycles are administered. In certain embodiments, greater than 10 cycles are administered.

In certain embodiments, one or more unit doses within a romidepsin dosing regimen may be administered via a route other than intravenous administration. In some embodiments, one or more doses may be administered orally. In certain embodiments, romidepsin is dosed orally in the range of 10 mg/m² to 300 mg/m². In certain embodiments, romidepsin is dosed orally in the range of 25 mg/m² to 100 mg/m². In certain embodiments, romidepsin is dosed orally in the range of 100 mg/m² to 200 mg/m². In certain embodiments, romidepsin is dosed orally in the range of 200 mg/m² to 300 mg/m². In certain embodiments, romidepsin is dosed orally at greater than 300 mg/m². In certain embodiments, romidepsin is dosed orally in the range of 50 mg/m² to 150 mg/m². In other embodiments, the oral dosage ranges from 25 mg/m² to 75 mg/m².

In certain embodiments, romidepsin is administered orally on a daily basis. In some embodiments, romidepsin is administered orally every other day. In still other embodiments, romidepsin is administered orally every third, fourth, fifth, or sixth day. In certain embodiments, romidepsin is administered orally every week. In certain embodiments, romidepsin is administered orally every other week.

In one embodiment, romidepsin is administered in a dose ranging from 10 mg/m² to 300 mg/m². In one embodiment, romidepsin is administered in a dose ranging from 15 mg/m² to 250 mg/m². In one embodiment, romidepsin is administered in a dose ranging from 20 mg/m² to 200 mg/m². In one embodiment, romidepsin is administered in a dose ranging from 25 mg/m² to 150 mg/m². In one embodiment, romidepsin is administered in a dose ranging from 25 mg/m² to 100 mg/m². In one embodiment, romidepsin is administered in a dose ranging from 25 mg/m² to 75 mg/m².

In one embodiment, romidepsin is administered orally on a daily basis. In one embodiment, romidepsin is administered orally every other day. In one embodiment, romidepsin is administered orally every third, fourth, fifth, or sixth day. In one embodiment, romidepsin is administered orally every week. In one embodiment, romidepsin is administered orally every other week.

In one embodiment, romidepsin is administered orally in a dose of 50 mg/m² on days 1, 8 and 15 of the 28 day cycle. In one embodiment, the cycle is repeated every 28 days.

In one embodiment, increasing doses of romidepsin are administered over the course of a cycle. In one embodiment, the dose of about 25 mg/m² followed by a dose of about 50 mg/m², followed by a dose of about 75 mg/m² is administered over a cycle.

In one embodiment, one cycle comprises the administration of from about 25 to about 150 mg/m² of romidepsin daily for three to four weeks and then one or two weeks of rest. In one embodiment, the number of cycles during which the treatment is administered to a patient will be from about one to about 40 cycles, or from about one to about 24 cycles, or from about two to about 16 cycles, or from about four to about three cycles.

In some embodiments, unit doses of romidepsin are within the range of about 0.5 mg/m² to about 28 mg/m² body surface area. In some embodiments, the range of about 6 to about 18 mg/m² is used. In some embodiments, the range is about 10 mg/m² to about 17 mg/m². In some embodiments, particular unit doses are 10 mg/m², 12 mg/m², 13 mg/m², 14 mg/m², and 15 mg/m².

In some embodiments, intravenous dosing regimens include daily dosing for 2 weeks, twice weekly dosing for 4 weeks, thrice weekly dosing for 4 weeks, and various other intermittent schedules (*e.g*., on days 1, 3, and 5; on days 4 and 10; on days 1, 8 and 15; on days 1 and 15; on days 5 and 12; or on days 5, 12, and 19 of 21 or 28 day cycles).

In some embodiments, romidepsin is administered in individual unit doses over 4 hours on days 1, 8, and 15, with courses repeating every 28 days. Often, several courses (*e.g*., at least 4, at least 6, or more) are administered. Indeed, instances have been reported of as many as 72 courses being administered. In some embodiments, individual unit doses are administered by 4 hour infusion.

### Accelerated Dosing for Romidepsin

Accelerated dosing regimens for romidepsin may be utilized, in which one or more individual unit doses is administered intravenously over a period of time that is less than or equal to about one hour. In some embodiments, one or more individual doses are administered intravenously over a period of time that is less than about 50 minutes, 40 minutes, 30 minutes, 20 minutes, or less. Any regimen that includes at least one unit dose administered over a period of time that is less than about one hour (60 minutes) may be considered an accelerated dosing regimen in accordance with the present disclosure.

In some embodiments, all unit doses within a regimen are administered intravenously over a time period that is less than or equal to about one hour. In some embodiments, only some of the unit doses within a regimen are administered over a time period that is less than or equal to about one hour. In some embodiments, one or more unit doses within a regimen are administered by a route other than intravenous administration (*e.g.,* oral, subcutaneous, nasal, topical, *etc.*)*.*

Accelerated dosing regimens of romidepsin can be administered without a significant increase in toxicity or adverse events, particularly in serious adverse events, as compared with a comparable regimen (*e.g*., an otherwise identical regimen) in which individual unit doses are administered intravenously over a 4-hour period. In one embodiment, accelerated dosing regimens can be administered without a significant increase in toxicity or adverse events, particularly in serious adverse events, as compared with a standard regimen of romidepsin administered by 4-hour intravenous infusion of a dose of about 6-14 mg/m² on days 1, 8, and 15 of a 28 day cycle.

In some embodiments, romidepsin is administered in an accelerated dosing regimen that is identical to a standard dosing regimen except that one or more unit doses is administered over a time period that is less than about 1 hour (*e.g*., rather than over a time period of about 4 hours).

As will be appreciated by one of skill in the art, the dosage, timing and/or routes of administration of particular unit doses of romidepsin may vary depending on the patient and condition being treated. In certain embodiments, the cycles are continued as long as the patient is responding. Therapy may be terminated once there is disease progression, a cure or remission is achieved, or side effects become intolerable. Adverse side effects may also call for lowering the dosage of romidepsin administered, or for adjusting the schedule by which doses are administered.

### PHARMACEUTICAL FORMULATIONS

In one embodiment, provided herein are pharmaceutical formulations, which comprise romidepsin, or a pharmaceutically acceptable salt or solvate thereof, as an active ingredient, in combination with one or more pharmaceutically acceptable carrier. In one embodiment, the pharmaceutical composition comprises at least one nonrelease controlling excipient or carrier. In one embodiment, the pharmaceutical composition comprises at least one release controlling and at least one nonrelease controlling excipient or carrier.

In certain embodiments, romidepsin used in the pharmaceutical compositions provided herein is in a solid form. Suitable solid forms include, but are not limited to, solid forms comprising romidepsin, and solid forms comprising salts of romidepsin. In certain embodiments, solid forms provided herein include polymorphs, solvates (including hydrates), and cocrystals comprising romidepsin and/or salts thereof. In certain embodiments, the solid form is an amorphous form of romidepsin, or a pharmaceutically acceptable salt or solvate thereof.

In one embodiment, the pharmaceutical compositions provided herein is formulated in various dosage forms for parenteral administration. In one embodiment, the pharmaceutical compositions provided herein is provided in a unit-dosage form or multiple-dosage form. A unit-dosage form, as used herein, refers to a physically discrete unit suitable for administration to human and animal subjects, and packaged individually as is known in the art. Each unit-dose contains a predetermined quantity of the active ingredient(s) sufficient to produce the desired therapeutic effect, in association with the required pharmaceutical carriers or diluents. Examples of a unit-dosage form include an ampoule, syringe, and individually packaged tablet and capsule. A unit-dosage form may be administered in fractions or multiples thereof. A multiple-dosage form is a plurality of identical unit-dosage forms packaged in a single container to be administered in segregated unit-dosage form. Examples of a multiple-dosage form include a vial, bottle of tablets or capsules, or bottle of pints or gallons.

In one embodiment, the composition is provided in 2 vials. One vial contains formulated romidepsin. The second vial contains a diluent.

In one embodiment, the pharmaceutical compositions provided herein is administered at once or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

In some embodiments, the composition is prepared by lyophilization from a solution. In particular embodiments, the composition is prepared by lyophilization from a solution of t-butanol and water. In some embodiments, the solvent is tert-butanol. In some embodiments, the solvent is a mixture of *tert*-butanol and water. In some embodiments, the solution is (60:40) (v/v) of t-butanol and water. In some embodiments, the pH adjustor is inorganic acid. In one embodiment, the inorganic acid is hydrochloric acid.

Diluents suitable for reconstitution of the formulation (or pharmaceutical composition) used in the methods provided herein include, but are not limited to, propylene glycol (PG), ethanol (EtOH) and water. In one embodiment, the solvent system is a combination of 30% EtOH, 45% PG and 25% water. In another embodiment, the solvent system is a combination of 40% EtOH, 30% PG and 30% water. In yet another embodiment, the solvent system is a combination of 13.4% EtOH, 53.3% PG and 33.3% water.

### Parenteral Administration

In one embodiment, the pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

In one embodiment, the pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (*see, e.g.,* Remington, The Science and Practice of Pharmacy, supra)*.*

In one embodiment, the pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

In one embodiment, suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (*e.g.,* polyethylene glycol 300 and polyethylene glycol 400), propylene glycol, glycerin, *N*-methyl-2-pyrrolidone, *N,N*-dimethylacetamide, and dimethyl sulfoxide.

In one embodiment, suitable antimicrobial agents or preservatives include, but are not limited to, phenols, cresols, mercurials, benzyl alcohol, chlorobutanol, methyl and propyl p-hydroxybenzoates, thimerosal, benzalkonium chloride (*e.g*., benzethonium chloride), methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including α-cyclodextrin, β-cyclodextrin, hydroxypropyl-β-cyclodextrin, sulfobutylether-β-cyclodextrin, and sulfobutylether 7-β-cyclodextrin (CAPTISOL®, CyDex, Lenexa, KS).

In one embodiment, the pharmaceutical compositions provided herein may be formulated for single or multiple dosage administration. The single dosage formulations are packaged in an ampoule, a vial, or a syringe. The multiple dosage parenteral formulations may contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

In one embodiment, the pharmaceutical compositions provided herein may be formulated as immediate or modified release dosage forms, including delayed-, sustained, pulsed-, controlled, targeted-, and programmed-release forms.

In one embodiment, the pharmaceutical compositions may be formulated as a suspension, solid, semi-solid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

In one embodiment, suitable inner matrixes include polymethylmethacrylate, polybutyl-methacrylate, plasticized or unplasticized polyvinylchloride, plasticized nylon, plasticized polyethylene terephthalate, natural rubber, polyisoprene, polyisobutylene, polybutadiene, polyethylene, ethylene-vinyl acetate copolymers, silicone rubbers, polydimethylsiloxanes, silicone carbonate copolymers, hydrophilic polymers, such as hydrogels of esters of acrylic and methacrylic acid, collagen, cross-linked polyvinyl alcohol, and cross-linked partially hydrolyzed polyvinyl acetate.

In one embodiment, suitable outer polymeric membranes include polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinyl chloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyloxyethanol copolymer, and ethylene/vinyl acetate/vinyl alcohol terpolymer.

### Combination Therapy

In some embodiments, romidepsin is administered in combination with one or more other pharmaceutical agents. In some embodiments, romidepsin is administered in combination with one or more other chemotherapeutic agents and/or in combination with one or more other pharmaceutical agents (*e.g*., pain relievers, anti-inflammatories, antibiotics, steroidal agents, anti-folates, kinase inhibitors, methyl transferase inhibitors, antibodies, *etc.*)*.*

In certain embodiments, romidepsin is administered in combination with one or more cytotoxic agents. Exemplary cytotoxic agents include, but are not limited to, gemcitabine, decitabine, and flavopiridol. In certain embodiments, romidepsin is administered in combination with one or more taxanes and/or one or more proteasome inhibitors. Exemplary proteasome inhibitors include, but are not limited to, bortezomib (VELCADE®), peptide boronates, salinosporamide A (NPI-0052), lactacystin, epoxomicin (Ac(Me)-Ile-Ile-Thr-Leu-EX), MG-132 (Z-Leu-Leu-Leu-al), PR-171, PS-519, eponemycin, aclacinomycin A, CEP-1612, CVT-63417, PS-341 (pyrazylcarbonyl-Phe-Leu-boronate), PSI (Z-Ile-Glu(OtBu)-Ala-Leu-al), MG-262 (Z-Leu-Leu-Leu-bor), PS-273 (MNLB), omuralide (*clasto*-lactacystin-β-lactone), NLVS (Nip-Leu-Leu-Leu-vinyl sulfone), YLVS (Tyr-Leu-Leu-Leu-vs), dihydroeponemycin, DFLB (dansyl-Phe-Leu-boronate), ALLN (Ac-Leu-Leu-Nle-al), 3,4-dichloroisocoumarin, 4-(2-aminoethyl)-benzenesulfonyl fluoride, TMC-95A, gliotoxin, EGCG ((-)-epigallocatechin-3-gallate), YU101 (Ac-hFLFL-ex), and combinations thereof.

In certain embodiments, romidepsin is administered in combination with one or more anti-folates. In some such embodiments, romidepsin is administered in combination with one or more of: folinic acid (leucovorin), methotrexate, pralatrexate, premextred, triazinate, or combinations thereof.

In certain embodiments, romidepsin is administered in combination with one or more kinase inhibitors (*e.g*., tyrosine kinase inhibitors). In some embodiments, romidepsin is administered in combination with one or more antibodies that act as a kinase inhibitor. In some embodiments, romidepsin is administered in combination with one or more of ABT-869, AC220, AZD7762, BIBW 2992, BMS-690154, CDKIAT7519, CYC116, ISIS3521, GSK690693, GSK-461364, MK-0457, MLN8054, MLN8237, MP470, ON 01910.Na, OSI-930, PHA-739358, R935788, SNS-314, TLN-232, XL147, XL228, XL281, XL418, or XL765.

In certain embodiments, romidepsin is administered in combination with one or more methyl transferase inhibitors.

In certain embodiments, romidepsin is administered in combination with one or more therapeutic antibodies. In some embodiments, the therapeutic antibodies include, but are not limited to, bevacizumab, cetuximab, dasatinib, erlotinib, geftinib, imatinib, lapatinib, nilotinib, panitumumab, pegaptanib, ranibizumab, sorafenib, sunitinib, trastuzumab, or any antibody that binds to an antigen bound by one of these moieties.

In some embodiments, romidepsin is administered in combination with an anti-inflammatory agent, pain reliever, anti-nausea medication, or anti-pyretic. Anti-inflammatory agents useful in the methods provided herein include, but are not limited to, aspirin, ibuprofen, and acetaminophen, *etc.*

In certain embodiments, romidepsin is administered in combination with a steroidal agent. In certain embodiments, romidepsin is administered in combination with a steroidal agent selected from the group consisting of alclometasone diproprionate, amcinonide, beclomethasone diproprionate, betamethasone, betamethasone benzoate, betamethasone diproprionate, betamethasone sodium phosphate, betamethasone sodium phosphate and acetate, betamethasone valerate, clobetasol proprionate, clocortolone pivalate, cortisol (hydrocortisone), cortisol (hydrocortisone) acetate, cortisol (hydrocortisone) butyrate, cortisol (hydrocortisone) cypionate, cortisol (hydrocortisone) sodium phosphate, cortisol (hydrocortisone) sodium succinate, cortisol (hydrocortisone) valerate, cortisone acetate, desonide, desoximetasone, dexamethasone, dexamethasone acetate, dexamethasone sodium phosphate, diflorasone diacetate, fludrocortisone acetate, flunisolide, fluocinolone acetonide, fluocinonide, fluorometholone, flurandrenolide, halcinonide, medrysone, methylprednisolone, methylprednisolone acetate, methylprednisolone sodium succinate, mometasone furoate, paramethasone acetate, prednisolone, prednisolone acetate, prednisolone sodium phosphate, prednisolone tebutate, prednisone, triamcinolone, triamcinolone acetonide, triamcinolone diacetate, triamcinolone hexacetonide, or combinations thereof. In some embodiments, romidepsin is administered in combination with dexamethasone.

In certain embodiments, romidepsin is administered in combination with an agent to treat gastrointestinal disturbances such as nausea, vomiting, and diarrhea. Such agents include, but are not limited to, anti-emetics, anti-diarrheals, fluid replacements, electrolyte replacements, *etc.*

In certain embodiments, romidepsin is administered in combination with electrolyte replacement or supplementation such as potassium, magnesium, and calcium. In certain embodiments, romidepsin is administered in combination with electrolyte replacement or supplementation such as potassium, and/or magnesium.

In certain embodiments, romidepsin is administered in combination with an anti-arrhythmic agent.

In certain embodiments, romidepsin is administered in combination with an agent that increases the production of platelets.

In certain embodiments, romidepsin is administered in combination with an agent to boost the production of blood cells. In certain embodiments, the agent is erythropoietin.

In some embodiments, romidepsin is administered in combination with an agent to prevent hyperglycemia.

In certain embodiments, romidepsin is administered with another HDAC or DAC inhibitor.

### Kits

In one embodiment, a kit comprises a dosage form of romidepsin, mannitol, and a diluent. Kits can further comprise a pharmacologically active derivative of romidepsin.

In other embodiments, kits can further comprise devices that are used to administer the active ingredients. Examples of such devices include, but are not limited to, syringes, and drip bags.

In one embodiment, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, alcohol, ethyl alcohol, dehydrated alcohol, polyethylene glycol, polypropylene glycol, and propylene glycol; and non-aqueous vehicles such as, but not limited to, corn oil, cottonseed oil, peanut oil, sesame oil, ethyl oleate, isopropyl myristate, and benzyl benzoate.

In one embodiment, the diluent is a combination of water, propylene glycol (PG), and ethanol (EtOH). In one embodiment, the ratio is 30% EtOH, 45% PG, and 25% of water. In another emdodiment, the ratio is 40% EtOH, 30% PG, and 30% of water. In yet another emdodiment, the ratio is 13.4% EtOH, 53.3% PG, and 33.3% of water.

In one embodiment, a kit contains a sterile, lyophilized powder in a single-use vial containing 10 mg of romidepsin and 20 mg of mannitol, and a second vial containing 30% EtOH, 45% PG, and 25% of water. In one embodiment, a kit contains a sterile, lyophilized powder in a single-use vial containing 10 mg of romidepsin and 20 mg of mannitol, and a second vial containing 40% EtOH, 30% PG, and 30% of water. In one embodiment, a kit contains a sterile, lyophilized powder in a single-use vial containing 10 mg of romidepsin and 20 mg of mannitol, and a second vial containing 13.4 EtOH, 53.3% PG, and 33.3% of water.

In another embodiment, a kit contains a sterile, lyophilized powder in a single-use vial containing 10 mg of romidepsin and 10 mg of mannitol, and a second vial containing 30% EtOH, 45% PG, and 25% of water. In one embodiment, a kit contains a sterile, lyophilized powder in a single-use vial containing 10 mg of romidepsin and 10 mg of mannitol, and a second vial containing 40% EtOH, 30% PG, and 30% of water. In one embodiment, a kit contains a sterile, lyophilized powder in a single-use vial containing 10 mg of romidepsin and 10 mg of mannitol, and a second vial containing 13.4 EtOH, 53.3% PG, and 33.3% of water.

### EXAMPLES

The following examples are provided by way of illustration, not limitation.

### Example 1. Preparation of Romidepsin Mannitol Formulation

Materials: Romidepsin (Sandoz GmBH); Mannitol (BDH); tert-butyl alcohol (TBA) (J.T. Baker); WFI, purified water or equivalent (Millipore MilliQ Advantage); and 1 N HCL (BDH).

Equipment: Mixer, Silverson, L5M-A; Lyophilizer, VirTis, Genesis 25EL; pH meter, Accumet Basic, AB15; Water bath capable of maintaining 30°C ± 2°C, Fisher Scientific Isotemp 3013 D; Vacuum filtration set, VWR cat. # 87006, batch W194402, containing 0.2 µM PES filter; 10 mL, glass type Flint 1, Gerresheimer, item # 80407100036, lot # 021325; 3-leg lyo (13x20) gry btyl slzd stoppers, Wheaton, W224100-202, lot # 1456376; 20 mm aluminum seals, blue, flip off, Wheaton, lot # 1461917-03; Balance, Shimadzu BW-4200D; Balance, Mettler Toledo, AT 261 Delta Range.

### Preparation of Compounding Solution (150 mL)

To prepare 200 mL of 60:40 (v/v), mixture of t-BA and purified water, 120 mL of t-BA and 80 mL of purified water were mixed. Approximaterly 95% of the required batch quantity of the t-BA/water mixture (or approximately 145 mL) were transferred to a suitable jacketed beaker connected to a water bath equilibrated at 30°C ± 2°C. The bath was temperature controlled. The required quantity of romidepsin (600 mg) was weighed in a weighing boat and transferred to the jacketed beaker containing the t-BA/water mixture. The content of the beaker was mixed at 2500-7500 rpm for 5 minutes or until romidepsin completely dissolves. The required quantity of mannitol (1200 mg) was weighed in a weighing boat and transferred to the jacketed beaker containing the t-BA/water/romidepsin solution. The content of the beaker was mixed at 2500-7500 rpm for 5 minutes or until mannitol completely dissolves. The pH of the solution was adjusted to a target pH 3.8 (range 3.6 - 4.0) with a predetermined amount of IN or 0.1 N HCl. The romidepsin/mannitol solution was further diluted to 150 mL by adding 60:40 (v/v) mixture of t-BA and purified water. The resulting mixture was mixed at 2500-7500 rpm for 5 minutes. The compounding tank was sealed, and the temperature was maintained at 28 to 32°C until sterile filtration.

### Sterile Filtration of Compounding Solution

The solution was sterilized by filtration using 0.2 µM sterilizing grade filter.

### Aseptic Filling of Vials for Drug Product

2.5 mL of the filtered romidepsin mannitol solution were filled into 10 mL lyophilization vials. Aseptic filling and stoppering of the sterile vials occurred under Class 100 conditions using an automated TL filling line. Process controls included defined weight checks of vials to verify accurate fill volume throughout the filling operation.

Immediately following filling of each vial, a sterile lyophilization stopper was partially seated in the vial and each tray of filled vials was moved to the loading area for the lyophilizer within the Class 100 aseptic area. Trays were immediately loaded onto precooled to 5°C shelves in the lyophilizer.

### Lyophilization

Vials containing romidepsin mannitol composition were lyophilized under aseptic conditions using a preprogrammed lyophilization cycle. A summary of the lyophilization cycle process for romidepsin mannitol formulation is provided in Table 1.

**Table 1.**

| Step | Lyophilization Cycle | | | |
|---|---|---|---|---|
| | Temp., °C | Time, min | Vacuum, mTorr | Ramp/Hold |
| Loading | 5 | N/A | N/A | Hold |
| 1 | 5 | 120 | N/A | Hold |
| 2 | -50 | 110 | N/A | Ramp |
| 3 | -50 | 240 | N/A | Hold |
| 4 | -18 | 65 | N/A | Ramp |
| 5 | -18 | 240 | N/A | Hold |
| 6 | -50 | 65 | N/A | Ramp |
| 7 | -50 | 240 | N/A | Hold |
| 8 | -50 | 10 | 90 | Hold |
| 9 | 0 | 100 | 90 | Ramp |
| 10 | 0 | 1500 | 90 | Hold |
| 11 | 40 | 80 | 90 | Ramp |
| 12 | 40 | 1080 | 190 | Hold |

In one embodiment, an additional step following step 12 (Table 1) includes drying the vials at the temperature of 50 °C up to 24 hours at the pressure of between 40 mTorr and 300 mTorr. In another embodiment, the additional step includes drying the vials at the temperature of 50 °C up to 48 hours at the pressure of between 40 mTorr and 300 mTorr.

In another embodiment, an additional step following step 12 (Table 1) includes drying the vials at the temperature of 60 °C up to 3 hours at the pressure of between 90 mTorr and 300 mTorr. In yet another embodiment, the additional step includes drying the vials at the temperature of 60 °C up to 6 hours at the pressure of between 90 mTorr and 300 mTorr. In another embodiment, the additional step includes drying the vials at the temperature of 60 °C up to 12 hours at the pressure of between 90 mTorr and 300 mTorr. In another embodiment, the additional step includes drying the vials at the temperature of 60 °C up to 24 hours at the pressure of between 90 mTorr and 300 mTorr. In another embodiment, the additional step includes drying the vials at the temperature of 60 °C up to 36 hours at the pressure of between 90 mTorr and 300 mTorr. In another embodiment, the additional step includes drying the vials at the temperature of 60 °C up to 48 hours at the pressure of between 90 mTorr and 300 mTorr.

In another embodiment, an additional step following step 12 (Table 1) includes drying the vials at the temperature of 70 °C up to 24 hours at the pressure of between 15 mTorr and 300 mTorr. In another embodiment, the additional step includes drying the vials at the temperature of 70 °C up to 48 hours at the pressure of between 15 mTorr and 300 mTorr..

In one embodiment, the romidepsin mannitol formulation was manufactured by drying additionaly for 36 hours at the temperature of 60 °C and the pressure of between 90 mTorr and 300 mTorr. A summary of the modified lyophilization process for romidepsin mannitol formulation is provided in Table 2.

**Table 2.**

| Step | Lyophilization Cycle | | | |
|---|---|---|---|---|
| | Temp., °C | Time, min | Vacuum, mTorr | Ramp/Hold |
| Loading | 5 | N/A | N/A | Hold |
| 1 | 5 | 120 | N/A | Hold |
| 2 | -50 | 110 | N/A | Ramp |
| 3 | -50 | 240 | N/A | Hold |
| 4 | -18 | 65 | N/A | Ramp |
| 5 | -18 | 240 | N/A | Hold |
| 6 | -50 | 65 | N/A | Ramp |
| 7 | -50 | 240 | N/A | Hold |
| 8 | -50 | 10 | 90 | Hold |
| 9 | 0 | 100 | 90 | Ramp |
| 10 | 0 | 1500 | 90 | Hold |
| 11 | 40 | 80 | 90 | Ramp |
| 12 | 40 | 1080 | 190 | Hold |
| 13 | 60 | 2160 | 90 | Hold |

In one embodiment, the romidepsin mannitol formulations manufactured using a regular lyophilization process and a modified lyophilization process were tested for residual t-butanol (t-BA) levels. The comparative analytical test results are shown in Table 3.

**Table 3.**

| **Analytical Test** | **Regular Lyophilization Process** | **Modified Lyophilization Process** |
|---|---|---|
| Appearance | White crumbly powder, no visible contaminants (n = 2) | White crumbly powder, no visible contaminants (n = 2) |
| Residual t-butanol, average ± SD | 0.211 ± 0.015 mg/vial (n = 3) | 0.065 ± 0.005 mg/vial (n = 3) |
| HPLC Identity | Confirmed (n = 1) | Confirmed (n = 1) |
| HPLC Assay | 92.4 % label claim (n = 1) | 92.3 % label claim (n = 1) |
| Impurities | 0.00 % (n = 1) | 0.00 % (n = 1) |
| KF moisture | 1.280 % (n = 1) | 0.334 % (n = 1) |

The results provided in Table 3 indicate that drying the romidepsin mannitol formulation additionally for 36 hours at the temperature of 60 °C and the pressure of 90 mTorr resulted in significantly lower levels of residual TBA in the composition.

In one embodiment, the lyophilization process for the romidepsin mannitol formulation was modified to reduce a primary drying time. In one embodiment, the primary drying time is 15.5 hours.

In one embodiment, the lyophilization process for the romidepsin mannitol formulation was modified to reduce drying time at the temperature of 60 °C. In one embodiment, the drying time at the temperature of 60 °C is 16 hours.

In one embodiment, the total freeze drying time of the lyophilization process is reduced. In one embodiment, the total freeze drying time is 2.4 days.

Following completion of the cycle, the vials were backfilled with sterile nitrogen, NF/EP, at atmospheric pressure and the stoppers were completely seated prior to opening the lyophilizer chamber. The trays were unloaded and transferred to the sealing area.

Vials containing compositions were sealed immediately following unloading from the lyophilization chamber. Each seal was imprinted with the composition lot number using a video jet printer incorporated into the automated sealing line. Seal inspection is performed every 15 minutes during the sealing operation.

Following sealing operations, romidepsin mannitol composition vials were inspected, labeled and packaged and appropriate process validation and/or evaluation was subsequently performed.

The resulting romidepsin mannitol formulation contained 4 mg/mL (10 mg/vial) of romidepsin and 8 mg/mL (20 mg/vial) of mannitol.

### Example 2. Diluent Optimization for Romidepsin/Mannitol Formulation

Materials used for the diluent optimization process included: romidepsin, lyophilized formulation (10 mg of romidepsin and 20 mg of mannitol in a 10 mL vial) for diluent screening and HPLC reproducibility study, propylene glycol (PG) (Sigma-Alorich), ethanol 200 proof (Decon Laboratories, INC.), sodium phosphate monobasic, monohydrate (J.T.Baker), sodium phosphate dibasic, anhydrous (J.T.Baker), 0.1N HCL solution (Fluka), and purified water or equivalent.

The diluent combinations were prepared by mixing PG, EtOH and water at different ratios, as shown in Figure 1. In one embodiment, the ratios for the solvent combinations are as follows: EtOH: 15% - 65%; water: 20% - 60%; and PG: 10% - 70%. Any solvent combination within the area covered with the black dots on Figure 1 can be used as diluent.

To investigate the solubility of the romidepsin mannitol formulation, 2 mL of the diluent mixture was added into the romidepsin mannitol lyophilized vial containing 10 mg of romidepsin and 20 mg of mannitol. The vial was hand shaked until the powder was totally dissolved or the time was reaching 3 minutes. The dissolution time was recorded by using a stopwatch. Diluents were either HPLC grade or ACS grade, unless stated otherwise. The diluent combinations used to test the dissolution of romidepsin mannitol formulation are shown in Table 4.

**Table 4.**

| **PG (mL)** | **PG (%)** | **EtOH (mL)** | **EtOH (%)** | **H2O (mL)** | **H2O (%)** |
|---|---|---|---|---|---|
| 2 | 18.2 | 8 | 72.7 | 1 | 9.1 |
| 2 | 16.7 | 8 | 66.7 | 2 | 16.7 |
| 2 | 15.4 | 8 | 61.5 | 3 | 23.1 |
| 2 | 14.3 | 8 | 57.1 | 4 | 28.6 |
| 2 | 13.3 | 8 | 53.3 | 5 | 33.3 |
| 2 | 12.5 | 8 | 50 | 6 | 37.5 |
| 2 | 10 | 8 | 40 | 10 | 50 |
| 2 | 6.7 | 8 | 26.7 | 20 | 66.7 |
| 4 | 36.4 | 6 | 54.5 | 1 | 9.1 |
| 4 | 33.5 | 6 | 50 | 2 | 16.7 |
| 4 | 30.8 | 6 | 46.2 | 3 | 23.1 |
| 4 | 28.6 | 6 | 42.9 | 4 | 28.6 |
| 4 | 26.7 | 6 | 40 | 5 | 33.3 |
| 4 | 25 | 6 | 37.5 | 6 | 37.5 |
| 4 | 23.5 | 6 | 35.3 | 7 | 41.2 |
| 4 | 20 | 6 | 30 | 10 | 50 |
| 6 | 54.5 | 4 | 36.4 | 1 | 9.1 |
| 6 | 50 | 4 | 33.3 | 2 | 16.7 |
| 6 | 46.2 | 4 | 30.8 | 3 | 23.1 |
| 6 | 42.9 | 4 | 28.6 | 4 | 28.6 |
| 6 | 40 | 4 | 26.7 | 5 | 33.3 |
| 6 | 37.5 | 4 | 25 | 6 | 37.5 |
| 6 | 35.3 | 4 | 23.5 | 7 | 41.2 |
| 6 | 30 | 4 | 20 | 10 | 50 |
| 8 | 72.7 | 2 | 18.2 | 1 | 9.1 |
| 8 | 66.7 | 2 | 16.7 | 2 | 16.7 |
| 8 | 61.5 | 2 | 15.4 | 3 | 23.1 |
| 8 | 57.1 | 2 | 14.3 | 4 | 28.6 |
| 8 | 53.5 | 2 | 13.3 | 5 | 33.3 |
| 8 | 50 | 2 | 12.5 | 6 | 37.5 |
| 8 | 40 | 2 | 10 | 10 | 50 |

The obtained results show that various diluent combinations containing PG, water, and EtOH can be used as diluents for the romidepsin mannitol formulation. The particular PG/EtOH/H₂O combinations (2 mL of PG/EtOH/H₂O) used to dissolve the romidepsin mannitol formulation are shown in Figure 2A.

### Example 3. Stability of Romidepsin Mannitol Formulation

The stability studies of romidepsin mannitol lyophilized formulation in various diluent systems were carried out by using a HPLC method. The parameters of the HPLC method were: flow rate: 1.0 mL/minute; injection volume: 4 µL; detectopm: 220 nm; column:Waters symmetry C18, 3.5 um, 4.6 x 150 mm; column temperature: 30°C; sample concentration: 5 mg/mL romidepsin; run time: 59 min; needle wash: diluent.

Two gradient programs were used: MPA: Phosphate buffer/acetonitrile 95/5; and MPB: Phosphate buffer/acetonitrile 40/60. HPLC gradients are shown in Table 5.

**Table 5.**

| **Time (mins)** | **% MPB** |
|---|---|
| 0 | 50 |
| 30 | 84 |
| 31 | 100 |
| 35 | 100 |
| 36 | 50 |
| 50 | 50 |

Mobile Phases (MP) were prepared by dissolving 20.52 g of 0.02M sodium phosphate monobasic monohydrate and 6.60 g of 0.05M sodium phosphate dibasic anhydrous in 2800 mL of water. Water was filled to 3000 mL, mixed, pH was measure, adjusted to 6.30 + 0.05 with IN NaOH or diluted phosphoric acid, if required and filtered.

For MPA 5/95 (V/V) acetonitrile/phosphate buffer solution: 1900 mL of buffer solution was thoroughly mixed with 100 mL of acetonitrile. For MPB 60/40 (V/V) acetonitrile/phosphate buffer solution: 800 mL of buffer solution were thoroughly mixed with 1200 mL of acetonitrile.

Stability of romidepsin mannitol formulation was tested in three diluent systems: diluent system A: 30% EtOH, 45% PG and 25% water; diluent system B: 40% EtOH, 30% PG and 30% water; and diluent system C: 13.4% EtOH, 53.3% PG and 33.3% water (Figure 1). The studies were performed at room temperature. The results of these studies are shown in Table 6.

**Table 6.**

| **Time (hrs)** | **% of initial AUC (Diluent A)** | **% of initial AUC (Diluent B)** | **% of initial AUC (Diluent C)** |
|---|---|---|---|
| Initial | 100.0 | 100.0 | 100.0 |
| 3 | 100.1 | 102.2 | 99.9 |
| 6 | 103.0 | 100.9 | 99.4 |
| 9 | 101.7 | 99.9 | 98.8 |
| 12 | 101.6 | 99.8 | 100.0 |
| 15 | 101.8 | 99.9 | 98.4 |
| 18 | 100.8 | 99.1 | 99.9 |
| 21 | 101.4 | 100.4 | 98.6 |
| 24 | 99.9 | 99.7 | 98.6 |

These results indicate that the romidepsin mannitol formulation was reasonably stable for at least 24 hours in all tested diluent systems.

## Claims

1. A lyophilized formulation comprising romidepsin and mannitol, wherein the ratio of romidepsin to mannitol is about 1 : 2 w/w, and wherein substantially all of the romidepsin is amorphous.

2. The formulation of claim 1, wherein the formulation is a unit dosage form.

3. The formulation of claim 1 or 2, wherein the amount of romidepsin is about 10 mg per vial.

4. The formulation of any of claims 1 to 3, wherein the amount of mannitol is about 20 mg per vial.

5. The formulation of claim 1 to 4, wherein said formulation is soluble in a solvent system consisting of propylene glycol (PG), ethanol (EtOH) and water, wherein the amount of PG is between 10% and 70%, and wherein the amount of EtOH is between 15% and 65%, and wherein the amount of water is between 20% and 60%.

6. The formulation of claim 5, wherein the ratio of solvents is 45 % PG, 30 % EtOH, and 25 % water.

7. The formulation of claim 5, wherein the ratio of solvents is 30 % PG, 40 % EtOH, and 30 % water.

8. The formulation of claim 5, wherein the ratio of solvents is 53.3 % PG, 13.4 % EtOH, and 33.3 % water.

9. The formulation of claim 5, wherein said formulation is soluble in the solvent system within 30 seconds, wherein the volume of the solvent system is 2 ml, and wherein the amount of formulation is up to 30 mg.

10. The formulation of claim 9, wherein the amount of romidepsin is about 10 mg and the amount of mannitol is about 20 mg.

11. A formulation of any of claims 1 to 10, for use in a method of treating cancer.

## Patentansprüche

1. Lyophilisierte Formulierung, umfassend Romidepsin und Mannit, wobei das Verhältnis von Romidepsin zu Mannit etwa 1:2 w/w beträgt und wobei im Wesentlichen das gesamte Romidepsin amorph ist.

2. Formulierung nach Anspruch 1, wobei die Formulierung eine Einheitsdosierungsform ist.

3. Formulierung nach Anspruch 1 oder 2, wobei die Menge von Romidepsin etwa 10 mg pro Ampulle beträgt.

4. Formulierung nach einem der Ansprüche 1 bis 3, wobei die Menge von Mannit etwa 20 mg pro Ampulle beträgt.

5. Formulierung nach Anspruch 1 bis 4, wobei die Formulierung in einem Lösungsmittelsystem löslich ist, das aus Propylenglykol (PG), Ethanol (EtOH) und Wasser besteht, wobei die Menge von PG zwischen 10 % und 70 % beträgt und wobei die Menge von EtOH zwischen 15 % und 65 % beträgt und wobei die Menge von Wasser zwischen 20 % und 60 % beträgt.

6. Formulierung nach Anspruch 5, wobei das Verhältnis der Lösungsmittel 45 % PG, 30 % EtOH und 25 % Wasser beträgt.

7. Formulierung nach Anspruch 5, wobei das Verhältnis der Lösungsmittel 30 % PG, 40 % EtOH und 30 % Wasser beträgt.

8. Formulierung nach Anspruch 5, wobei das Verhältnis der Lösungsmittel 53,3 % PG, 13,4 % EtOH und 33,3 % Wasser beträgt.

9. Formulierung nach Anspruch 5, wobei die Formulierung innerhalb von 30 Sekunden in dem Lösungsmittelsystem löslich ist, wobei das Volumen des Lösungsmittelsystems 2 ml beträgt und wobei die Menge der Formulierung bis zu 30 mg beträgt.

10. Formulierung nach Anspruch 9, wobei die Menge von Romidepsin etwa 10 mg beträgt und die Menge von Mannit etwa 20 mg beträgt.

11. Formulierung nach einem der Ansprüche 1 bis 10 zur Verwendung in einem Verfahren zum Behandeln von Krebs.

## Revendications

1. Formulation lyophilisée comprenant de la romidepsine et du mannitol, dans laquelle le rapport de la romidepsine au mannitol est d'environ 1:2 p/p, et dans laquelle sensiblement toute la romidepsine est amorphe.

2. Formulation selon la revendication 1, dans laquelle la formulation est une forme posologique unitaire.

3. Formulation selon la revendication 1 ou 2, dans laquelle la quantité de romidepsine est d'environ 10 mg par flacon.

4. Formulation selon l'une quelconque des revendications 1 à 3, dans laquelle la quantité de mannitol est d'environ 20 mg par flacon.

5. Formulation selon la revendication 1 à 4, dans laquelle ladite formulation est soluble dans un système de solvant constitué de propylène glycol (PG), d'éthanol (EtOH) et d'eau, dans laquelle la quantité de PG est comprise entre 10 % et 70 %, et dans laquelle la quantité d'EtOH est comprise entre 15 % et 65 %, et dans laquelle la quantité d'eau est comprise entre 20 % et 60 %.

6. Formulation selon la revendication 5, dans laquelle le rapport des solvants est de 45 % de PG, 30 % d'EtOH et 25 % d'eau.

7. Formulation selon la revendication 5, dans laquelle le rapport des solvants est de 30 % de PG, 40 % d'EtOH et 30 % d'eau.

8. Formulation selon la revendication 5, dans laquelle le rapport des solvants est de 53,3 % de PG, 13,4 % d'EtOH et 33,3 % d'eau.

9. Formulation selon la revendication 5, dans laquelle ladite formulation est soluble dans le système de solvant en 30 secondes, dans laquelle le volume du système de solvant est de 2 ml, et dans laquelle la quantité de formulation va jusqu'à 30 mg.

10. Formulation selon la revendication 9, dans laquelle la quantité de romidepsine est d'environ 10 mg et la quantité de mannitol est d'environ 20 mg.

11. Formulation selon l'une quelconque des revendications 1 à 10, destinée à être utilisée dans un procédé de traitement du cancer.
